# EUROPÄISCHE PATENTSCHRIFT

(11) **EP 4 448 048 B1**
(45) Veröffentlichungstag und Bekanntmachung des Hinweises auf die Patenterteilung: **11.02.2026**
(21) Anmeldenummer: 22839673.5
(22) Anmeldetag: 15.12.2022
(51) Int. Cl.: A61M 3/02, A61B 5/00, A61B 17/3203, A61B 34/00, A61B 34/32, A61M 1/00, A61B 34/10

(54) **ROBOTERSYSTEM FÜR DIE WUNDREINIGUNG**
ROBOTIC SYSTEM FOR WOUND CLEANING
SYSTÈME ROBOTIQUE POUR NETTOYAGE DE PLAIE

(30) Priorität: 15.12.2021 DE 102021133193
(43) Veröffentlichungstag der Anmeldung: 23.10.2024
(73) Patentinhaber: Deutsches Zentrum für Luft- und Raumfahrt e.V., 53227 Bonn (DE); Albert-Ludwigs-Universität Freiburg, 79098 Freiburg (DE)
(72) Erfinder: BAHLS, Thomas, 86947 Weil (DE); MIERNIK, Arkadiusz, 79110 Freiburg (DE); SCHÖB, Dominik, 79114 Freiburg (DE)
(74) Vertreter: dompatent
(86) Internationale Anmeldenummer: PCT/EP2022/086177
(87) Internationale Veröffentlichungsnummer: WO 2023/111190

(56) Entgegenhaltungen:
- WO-A1-2018/005449
- CN-A- 109 330 803
- CN-A- 110 236 736
- US-A1- 2008 033 410
- US-A1- 2015 335 343
- US-A1- 2020 023 116
- US-A1- 2020 261 629

## Beschreibung

Die Erfindung betrifft ein Robotersystem für die Wundreinigung.

Aus dem Stand der Technik ist es bekannt, eine Wundauswaschung mit einem medizinischen Wasserstrahl sowie Wundebridement manuell durch eine medizinische Fachkraft durchzuführen. Eine große Herausforderung besteht hierbei darin, möglichst die gesamte Wundoberfläche zu erfassen und deren Behandlung mit einer konstant bleibenden Qualität durchzuführen, um das Risiko einer Reinfektion zu minimieren. Eine visuelle Kontrolle durch die medizinische Fachkraft ist oft schwierig, da sich häufig ein bereits bearbeitetes Flächenstück nicht wesentlich von einem unbearbeiteten unterscheidet.

Weiterhin ist es bekannt, eine Wundauswaschung automatisiert unter Verwendung eines Roboterarm durchzuführen. Hierbei kann eine gleichmäßigere Abdeckung der Wundoberfläche erreicht werden. Allerdings weisen die Zieloberflächen oftmals eine inhomogene Beschaffenheit auf, sodass das Ergebnis dennoch häufig unbefriedigend ist.

Informationen zum Stand der Technik können der folgenden Veröffentlichung entnommen werden:
Robot Guided Waterjet Surgery Dissertation, Thomas Bahls 2020 TU München

CN 110236736 A beschreibt ein Robotersystem für die Wundreinigung mit einem Roboterarm, an dem eine Düse zur Ausgabe einer Reinigungsflüssigkeit angebracht ist.

US 2015/335343 A1 beschreibt ebenfalls ein System zur Wundreinigung, wobei verschiedene Sensoren offenbart werden, mithilfe derer gesundes von nekrotischem Gewebe unterschieden werden kann.

Aufgabe der Erfindung ist es, ein Robotersystem für die Wundreinigung bereit zu stellen, das ein verbessertes Reinigungsergebnis ermöglicht.

Die Lösung der Aufgabe erfolgt erfindungsgemäß durch die Merkmale des Anspruchs 1.

Das erfindungsgemäße Robotersystem für die Wundreinigung weist einen Roboterarm auf, an dem eine Düse zur Ausgabe einer Reinigungsflüssigkeit angebracht ist. Hierbei kann es sich beispielsweise um Wasser handeln, das zusätzlich ein therapeutisches Mittel für eine verbesserte Reinigung und/ oder ein lokales Anästhetikum aufweisen kann. Zusätzlich kann die Reinigungsflüssigkeit weitere Moleküle aufweisen, die sowohl mechanisch als auch chemisch mit der zu reinigenden Oberfläche interagieren.

Das Robotersystem weist eine Steuervorrichtung zum Steuern des Roboterarms derart auf, dass durch die Düse bestimmte Positionen auf der Wundoberfläche gezielt gereinigt werden.

Weiterhin weist das Robotersystem mindestens einen Sensor zum Erfassen der Güte des Reinigungsergebnisses auf.

Das Robotersystem umfasst ferner ein Regelsystem zum Ansteuern von Positionen auf der Wundoberfläche durch die Düse, bei denen die Güte des Reinigungsergebnisses einen gewünschten Schwellwert nicht erreicht hat.

Durch das erfindungsgemäße Robotersystem kann eine gleichmäßig hohe Reinigungsqualität über die gesamte Wundoberfläche erreicht werden. Dies erfolgt in sehr zuverlässiger Weise, da die Bewertung der Reinigungsqualität automatisiert unter Verwendung eines Sensors und nicht durch eine medizinische Fachkraft erfolgt.

Es ist bevorzugt, dass der mindestens eine Sensor ein Wärmebildsensor zum Erfassen der Durchblutung der Wundoberfläche ist. Dies kann beispielsweise durch einen berührungslosen Infrarotsensor erfolgen.

Alternativ oder zusätzlich kann der mindestens eine Sensor ein Hyperspektralsensor zum Erfassen der spektralen Signatur von unerwünschten Belägen auf der Wundoberfläche sein. Hierbei wird die spektrale Signatur der Wundoberfläche direkt erfasst, sodass bestimmte Beläge z.B. Bakterienkolonien in einem bestimmten Spektrum sichtbar gemacht werden können.

Alternativ oder zusätzlich kann der mindestens eine Sensor ein Sensor zu Analyse der chemischen Zusammensetzung des abgetragenen Materials sein, das ihm über eine Absaugvorrichtung zugeführt wird. Diese Absaugvorrichtung kann Teil des Robotersystems sein. Beispielsweise kann das abgesaugte Medium im Hinblick auf therapierelevante Merkmale wie zum Beispiel Proteine, Saccharide sowie bekannte Bakterienarten oder deren Zellbestandteile untersucht werden. Auch kann eine Analyse auf Hämoglobin im abgesaugten Medium erfolgen.

Alternativ oder zusätzlich kann der mindestens eine Sensor ein Widerstandssensor zum Erfassen des elektrischen Widerstands der Wundoberfläche sein. Wird beispielsweise mit einer Kochsalzlösung gespült so ist deren spezifischer Widerstand bekannt. Verunreinigungen auf der Wundoberfläche führen zu einer Änderung des Widerstandes, sodass hieraus die Qualität des Reinigungsergebnisses abgeleitet werden kann.

Alternativ oder zusätzlich kann der mindestens eine Sensor ein MEMS-Sensor sein. Dieser kann als ein MEMS-Sensor basierter Mikroreaktor ausgebildet sein, dem über die Absaugvorrichtung abgesaugte Flüssigkeit zugeführt wird. Der Mikroreaktor kann sodann in Echtzeit beispielsweise die chemische Zusammensetzung der abgesaugten Flüssigkeit analysieren, um die Güte des Reinigungsergebnisses beurteilen zu können. Auch ist es möglich, die abgesaugte Flüssigkeit auf Bakterien oder deren Zellbestandteile, Proteine, Saccharide und/ oder Hämoglobin im Mikroreaktor zu analysieren.

Bei allen beschriebenen Sensorarten erfolgt eine Analyse des Reinigungsergebnisses in Echtzeit, sodass durch das Regelsystem Positionen auf der Wundoberfläche gezielt angesteuert werden, bei denen die Güte des Reinigungsergebnisses einen gewünschten Schwellwert nicht erreicht hat.

In allen Ausführungsformen der Erfindung ist es möglich eine Datenbank zu erstellen, welche es ermöglicht, die Beläge anhand ihres Spektrums zu identifizieren. Hierzu wird parallel zur spektralen Analyse eine chemische Analyse durchgeführt, bis eine ausreichende Datenbasis aufgebaut wurde

Das erfindungsgemäße Robotersystem umfasst ferner bevorzugt eine Geometrie-Erfassungsvorrichtung zur Erfassung der Geometrie der Wundoberfläche. Diese kann beispielsweise dreidimensional vermessen werden. Das Robotersystem umfasst ferner eine Modelliervorrichtung zum Modellieren der Wundoberfläche, wobei beim Modellieren ein Aufteilen der Wundoberfläche in eine Vielzahl von Teilflächen erfolgt. Die Steuervorrichtung ist ausgebildet zum gezielten Reinigen von Teilflächen der Wundoberflächen, bei denen die Güte des Reinigungsergebnisse einen gewünschten Schwellwert nicht erreicht hat.

Bevorzugt umfasst die Geometrieerfassungsvorrichtung ein optisches System, insbesondere ein structured light system, ein stereo vision system, ein laserbasiertes Triangulationssystem oder ein kinesthetic sampling system.

Das erfindungsgemäße Robotersystem umfasst ferner bevorzugt eine Zugabevorrichtung zum Zugeben eines therapeutischen Mittels zur Spülflüssigkeit. Hierbei kann die Konzentration des therapeutischen Mittels an den Positionen auf der Wundoberfläche erhöht werden, an denen die Güte des Reinigungsergebnisses einen gewünschten Schwellwert nicht erreicht hat.

In dem in der Beschreibungseinleitung genannten Stand der Technik ("Robot Robot Guided Waterjet Surgery Dissertation") ist hierzu beschrieben, wie ausgehend von der Zielfläche und der verwendeten dissection trajectory des Wasserstrahls (welche die Geometrie des aktuierten Wasserstrahlapplikators beschreibt) eine Trajektorie berechnet werden kann, welche zumindest geometrisch eine hohe Abdeckung ermöglicht.

In allen Ausführungsformen der Erfindung sollte sichergestellt werden, dass der Wasserstrahl immer senkrecht bzw. immer in einem gleichbleibenden Winkel auf die Wundoberfläche trifft.

Weiterhin ist die Steuerung bevorzugt ausgebildet zum Verringern des Abstandes der Düse zur Wundoberfläche und/ oder zur Erhöhung des Wasserdrucks an Positionen auf der Wundoberfläche, bei denen die Güte des Reinigungsergebnisses einen gewünschten Schwellwert nicht erreicht hat.

Das Ergebnis der Behandlung kann überprüft und in qualitativer sowie quantitativer Form visualisiert und alphanumerisch abgebildet werden.

Die Wundreinigung kann ferner mit fluoreszenzpartikeldotierter Flüssigkeit zur späteren optischen Prüfung der Oberfläche erfolgen.

Weiterhin kann die Wundreinigung mit Flüssigkeit und abrasiven Partikeln (z.B. Mikroplastik, Silizium, Kalk) erfolgen.

Im Folgenden wird eine bevorzugte Ausführungsform der Erfindung anhand einer Figur erläutert.

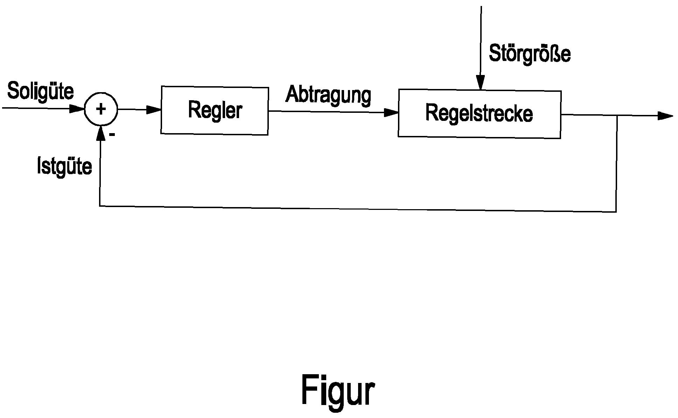

Die Figur zeigt ein Regelsystem zum Regeln des erfindungsgemäßen Robotersystems. Die von dem mindestens einen Sensor IST-Güte des Reinigungsergebnisses wird von der SOLL-Güte subtrahiert, sodass dem Regler ein Abweichungswert zugeführt wird

Die Abtragung kann hierbei individuell für verschiedene Positionen auf der Wundoberfläche geregelt werden, sodass individuelle Störgrößen berücksichtigt werden können. Eine Regelung der Abtragung kann über eine Veränderung des Drucks und/ oder der Dauer der Wasserstrahlreinigung erfolgen. Voraussetzungen hierbei sind eine gleichbleibende Düsengeometrie, ein kontinuierliches Absaugen sowie eine Verwendung im laminaren Bereich.

## Patentansprüche

1. Robotersystem für die Wundreinigung, mit
einem Roboterarm, an dem eine Düse zur Ausgabe einer Reinigungsflüssigkeit angebracht ist,
einer Steuervorrichtung zum Steuern des Roboterarms derart, dass durch die Düse bestimmte Positionen auf der Wundoberfläche gezielt gereinigt werden,
mindestens einem Sensor zum Erfassen der Güte des Reinigungsergebnisses,
einem Regelsystem zum Ansteuern von Positionen auf der Wundoberfläche, bei denen die Güte des Reinigungsergebnisses einen gewünschten Schwellwert nicht erreicht hat,
wobei eine Analyse des Reinigungsergebnisses in Echtzeit erfolgt, sodass durch das Regelsystem Positionen auf der Wundoberfläche gezielt angesteuert werden, bei denen die Güte des Reinigungsergebnisses einen gewünschten Schwellwert nicht erreicht hat.

2. Robotersystem nach Anspruch 1, **dadurch gekennzeichnet, dass** der mindestens erste Sensor ein Wärmebildsensor zum Erfassen der Durchblutung der Wundoberfläche ist.

3. Robotersystem nach Anspruch 1, **dadurch gekennzeichnet, dass** der mindestens eine Sensor ein Hyperspektralsensor zum Erfassen der spektralen Signatur von unerwünschten Belägen auf der Wundoberfläche ist.

4. Robotersystem nach Anspruch 1, **dadurch gekennzeichnet, dass** der mindestens eine Sensor ein Sensor zur Analyse der chemischen Zusammensetzung des abgetragenen Materials ist, das ihm über eine Absaugvorrichtung zugeführt wird.

5. Robotersystem nach Anspruch 1, **dadurch gekennzeichnet, dass** der mindestens eine Sensor ein Widerstandssensor zum Erfassen des elektrischen Widerstands der Wundoberfläche ist.

6. Robotersystem nach Anspruch 1, **dadurch gekennzeichnet, dass** der mindestens eine Sensor ein MEMS-Sensor ist.

7. Robotersystem nach Anspruch 1, **dadurch gekennzeichnet, dass** das Robotersystem eine Geometrie-Erfassungsvorrichtung zur Erfassung der Geometrie der Wundoberfläche aufweist,
wobei das Robotersystem ferner eine Modelliervorrichtung zum Modellieren der Wundoberfläche aufweist,
wobei beim Modellieren ein Aufteilen der Wundoberfläche in eine Vielzahl von Teilflächen erfolgt
und die Steuervorrichtung ausgebildet ist zum gezielten Reinigen von Teilflächen der Wundoberfläche, bei denen die Güte des Reinigungsergebnisses einen gewünschten Schwellwert nicht erreicht hat.

8. Robotersystem nach Anspruch 7, **dadurch gekennzeichnet, dass** die Geometrieerfassungsvorrichtung ein optisches System, insbesondere ein structured light system, ein stereo vision system, ein laserbasiertes Triangulationssystem oder ein kinesthetic sampling system umfasst.

9. Robotersystem nach Anspruch 1 bis 8, **dadurch gekennzeichnet**, durch eine Zugabevorrichtung zum Zugeben von einem therapeutischen Mittel zur Spülflüssigkeit,
wobei die Konzentration des therapeutischen Mittels an den Positionen auf der Wundoberfläche erhöht wird, an denen die Güte des Reinigungsergebnisses einen gewünschten Schwellwert nicht erreicht hat.

10. Robotersystem nach Anspruch 1 bis 9, **dadurch gekennzeichnet, dass** die Steuerung ausgebildet ist zum Verringern des Abstandes der Düse zur Wundoberfläche und/ oder zur Erhöhung des Wasserdrucks bei Positionen auf der Wundoberfläche, bei denen die Güte des Reinigungsergebnisses einen gewünschten Schwellwert nicht erreicht hat.

## Claims

1. A robotic system for wound cleaning, comprising
a robotic arm to which a nozzle for dispensing a cleaning liquid is attached,
a control device for controlling the robotic arm in such a way that the nozzle cleans specific positions on the wound surface in a targeted manner,
at least one sensor for detecting the quality of the cleaning result,
a control system for controlling positions on the wound surface where the quality of the cleaning result has not reached a desired threshold value,
wherein the cleaning result is analyzed in real time so that the control system specifically targets positions on the wound surface where the quality of the cleaning result has not reached a desired threshold value.

2. The robotic system according to claim 1, **characterized in that** the at least first sensor is a thermal image sensor for detecting the blood circulation of the wound surface.

3. The robotic system according to claim 1, **characterized in that** the at least one sensor is a hyperspectral sensor for detecting the spectral signature of undesired coatings on the wound surface.

4. The robotic system according to claim 1, **characterized in that** the at least one sensor is a sensor for analyzing the chemical composition of the removed material, which is fed thereto via a suction device.

5. The robotic system according to claim 1, **characterized in that** the at least one sensor is a resistance sensor for detecting the electrical resistance of the wound surface.

6. The robotic system according to claim 1, **characterized in that** the at least one sensor is a MEMS sensor.

7. The robotic system according to claim 1, **characterized in that** the robotic system has a geometry detection device for detecting the geometry of the wound surface, wherein the robotic system further comprises a modeling device for modeling the wound surface,
wherein the wound surface is divided into a plurality of partial surfaces during modeling
and wherein the control device is configured for targeted cleaning of partial areas of the wound surface where the quality of the cleaning result has not reached a desired threshold value.

8. The robotic system according to claim 7, **characterized in that** the geometry detection device comprises an optical system, in particular a structured light system, a stereo vision system, a laser-based triangulation system or a kinesthetic sampling system.

9. The robotic system according to claim 1 to 8, **characterized by** an addition device for adding a therapeutic agent to the cleaning liquid,
whereby the concentration of the therapeutic agent is increased at the positions on the wound surface where the quality of the cleaning result has not reached a desired threshold value.

10. The robotic system according to claim 1 to 9, **characterized in that** the controller is configured to reduce the distance of the nozzle to the wound surface and/or to increase the water pressure at positions on the wound surface at which the quality of the cleaning result has not reached a desired threshold value.

## Revendications

1. Système robotisé pour le nettoyage des plaies, avec
un bras robotisé sur lequel est disposée une buse pour la distribution d'un liquide de nettoyage,
un dispositif de commande pour commander le bras robotisé de telle sorte que des positions déterminées sur la surface de la plaie sont nettoyées de manière ciblée par la buse,
au moins un capteur pour acquérir la qualité du résultat du nettoyage,
un système de régulation pour viser des positions sur la surface de la plaie où la qualité du résultat du nettoyage n'a pas atteint un seuil souhaité,
où une analyse du résultat du nettoyage a lieu en temps réel, de sorte que des positions sur la surface de la plaie où la qualité du résultat du nettoyage n'a pas atteint un seuil souhaité sont visées de manière ciblée par le système de régulation.

2. Système robotisé selon la revendication 1, **caractérisé en ce que** le au moins premier capteur est un capteur d'imagerie thermique pour acquérir le flux sanguin de la surface de la plaie.

3. Système robotisé selon la revendication 1, **caractérisé en ce que** le au moins un capteur est un capteur hyperspectral pour acquérir la signature spectrale de dépôts indésirables sur la surface de la plaie.

4. Système robotisé selon la revendication 1, **caractérisé en ce que** le au moins un capteur est un capteur pour l'analyse de la composition chimique du matériau prélevé qui lui est envoyé par un dispositif d'aspiration.

5. Système robotisé selon la revendication 1, **caractérisé en ce que** le au moins un capteur est un capteur de résistance pour acquérir la résistance électrique de la surface de la plaie.

6. Système robotisé selon la revendication 1, **caractérisé en ce que** le au moins un capteur est un capteur MEMS.

7. Système robotisé selon la revendication 1, **caractérisé en ce que** le système robotisé présente un dispositif d'acquisition de géométrie pour acquérir la géométrie de la surface de la plaie,
où le système robotisé présente en outre un dispositif de modélisation pour la modélisation de la surface de la plaie,
où lors de la modélisation, une division de la surface de la plaie en une multiplicité de surfaces partielles a lieu
et le dispositif de commande est configuré pour le nettoyage ciblé de surfaces partielles de la surface de la plaie pour lesquelles la qualité du résultat du nettoyage n'a pas atteint un seuil souhaité.

8. Système robotisé selon la revendication 7, **caractérisé en ce que** le dispositif d'acquisition de géométrie comprend un système optique, notamment un système à lumière structurée, un système de vision stéréoscopique, un système de triangulation à base de laser ou un système d'échantillonnage kinesthésique.

9. Système robotisé selon les revendications 1 à 8, **caractérisé par** un dispositif d'addition pour ajouter un agent thérapeutique au liquide de rinçage,
où la concentration de l'agent thérapeutique est augmentée aux positions sur la surface de la plaie où la qualité du résultat du nettoyage n'a pas atteint un seuil souhaité.

10. Système robotisé selon les revendications 1 à 9, **caractérisé en ce que** la commande est configurée pour réduire la distance de la buse à la surface de la plaie et/ou pour augmenter la pression de l'eau aux positions sur la surface de la plaie où la qualité du résultat du nettoyage n'a pas atteint un seuil souhaité.
